# EUROPÄISCHE PATENTSCHRIFT

(11) **EP 0 842 278 B1**
(45) Veröffentlichungstag und Bekanntmachung des Hinweises auf die Patenterteilung: **09.11.2005**
(21) Anmeldenummer: 96915989.6
(22) Anmeldetag: 10.06.1996
(51) Int. Cl.: C12N 15/55, C12N 9/22, C12N 15/70, C12N 1/21, C07K 16/40, A61K 38/46, A61K 48/00, A61K 39/395, G01N 33/573, C12Q 1/68

(54) **PROTEIN MIT DNASE-AKTIVITÄT**
DNASE-ACTIVE PROTEIN
PROTEINE A ACTIVITE DE DESOXYRIBONUCLEASE

(30) Priorität: 09.06.1995 DE 19521046
(43) Veröffentlichungstag der Anmeldung: 20.05.1998
(73) Patentinhaber: Coy, Johannes Dr., 69221 Dossenheim (DE)
(72) Erfinder: ZENTGRAF, Hanswalter, D-69115 Heidelberg (DE); POUSTKA, Annemarie, D-69120 Heidelberg (DE); COY, Johannes, D-63762 Grossostheim (DE); VELHAGEN, Iris, D-68723 Schwetzingen (DE)
(74) Vertreter: Rudolph, Ulrike
(86) Internationale Anmeldenummer: PCT/DE1996/001016
(87) Internationale Veröffentlichungsnummer: WO 1996/041887

(56) Entgegenhaltungen:
- WO-A-90/07572
- EMBL Datenbank Eintrag HS068461 Zugriffsnummer U06846; 4 Juni 1995 BIUNNO I. ET AL.:'Identification of a novel gene (XIB) in the human Xq28 region' XP002015292
- CELL DEATH AND DIFFERENTIATION, Bd. 3, Nr. 2, 1.April 1996, Seiten 199-206, XP000603900 JOHANNES F. COY ET AL.: "Isolation, differential splicing amd protein expression of a DNAse on the human X chromosome"
- GENE (1996), 168(2), 267-70 CODEN: GENED6;ISSN: 0378-1119, 1996, XP002015290 PERGOLIZZI, ROSSANA ET AL: "Cloning of a gene encoding a DNase I-like endonuclease in the human Xq28 region"
- HUMAN MOLECULAR GENETICS, Bd. 4, Nr. 9, September 1995, OXFORD GB, Seiten 1557-1564, XP002015291 JULIA E. PARRISH ET AL.: "A muscle-specific DNAse I-like gene in human Xq28"

## Beschreibung

Die vorliegende Erfindung betrifft ein Protein mit DNase-Aktivität, eine ein solches kodierende DNA und ein Verfahren zur Herstellung eines solchen. Ferner betrifft die Erfindung die Verwendung der DNA und des Proteins sowie gegen das Protein gerichtete Antikörper.

Es ist bekannt, daß viele Zellen einen programmierten Zelltod erleiden. Dieser Zelltod wird mit Apoptose bezeichnet. Er findet sich z.B. bei der Organentwicklung und Metamorphose, der Gewebeatrophie und Tumorregression. Apoptose ist mit einer Kondensation des Cytoplasmas, einem Verlust von Plasmamembranvilli, einer Segmentation des Kerns und insbesondere einem extensiven Abbau chromosomaler DNA verbunden. Letzteres zeigt sich darin, daß in apoptotischen Zellen eine "Leiter" von DNA-Fragmenten, insbesondere der Größe von mehr als 600 kb, 50-300 kb und 50 kb, vorliegt. Bisher ist nicht bekannt, welche Mechanismen für den Abbau der chromosonalen DNA verantwortlich sind. Dies wäre aber notwendig, um Apoptose besser verstehen und gegebenenfalls Maßnahmen für oder gegen sie ergreifen zu können.

WO-A-900 75 72 beschreibt eine menschliche DNase, die eine Homologie von ca. 55% zur DNase der gegenwärtigen Anmeldung aufweist.

Der vorliegenden Erfindung liegt somit die Aufgabe zugrunde, ein Mittel bereitzustellen, mit dem der Abbau chromosomaler DNA in apoptotischen Zellen untersucht werden kann.

Erfindungsgemäß wird dies durch die Bereitstellung der Gegenstände in den Patentansprüchen erreicht.

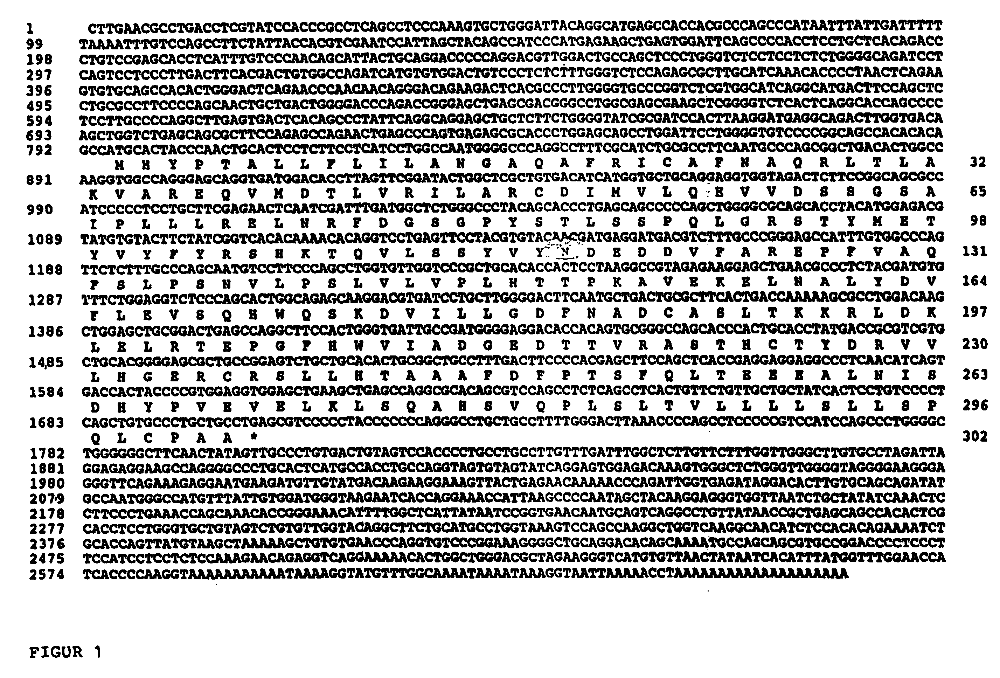

Gegenstand der vorliegenden Erfindung ist somit ein Protein mit DNase-Aktivität, das die Aminosäuresequenz von Fig. 1 oder ein funktionelles Derivat oder Fragment davon umfaßt, wobei dieses Derivat oder Fragment (i) die Aminosäuresequenz von Fig. 1 mit Addition und/oder Substitution und/oder Deletion einer oder mehrerer Aminosäuren aufweist, (ii) von einer DNA codiert wird, die mit der DNA von Fig 1 bei 20°C unter dem Schmelzpunkt der DNA hybridisiert und (iii) eine DNA ase Aktivität aufweist. Der Ausdruck "DNase-Aktivität" weist daraufhin, daß das Protein einzel- und/oder doppelsträngige DNA schneiden kann.

Der Ausdruck "funktionelles Derivat oder Fragment" umfaßt jegliches Derivat oder Fragment der Aminosäuresequenz von Fig. 1, das eine DNase-Aktivität aufweist. Auch kann die Aminosäuresequenz von Fig. 1 Additionen Substitutionen und/oder Deletionen von ein oder mehreren Aminosäuren aufweisen, was auch für die funktionellen Derivate oder Fragmente davon gilt.

Ein weiterer Gegenstand der vorliegenden Erfindung ist eine für ein vorstehendes Protein kodierende Nukleinsäure. Dies kann eine RNA oder eine DNA sein. Letztere kann z.B. eine genomische DNA oder eine cDNA sein. Bevorzugt ist eine DNA, die folgendes umfaßt:
(a) die DNA von Fig. 1
(b) eine mit der DNA von (a) hybridisierende DNA, oder
(c) eine mit der DNA von (a) oder (b) über den degenerierten genetischen Code verwandte DNA.

Der Ausdruck "hybridisierende DNA" weist auf eine DNA hin, die unter üblichen Bedingungen, insbesondere bei 20°C unter dem Schmelzpunkt der DNA, mit einer DNA von (a) hybridisiert.

Die DNA von Fig. 1 wurde bei der DSM (Deutsche Sammlung von Mikroorganismen und Zellkulturen) als JFC4 unter DSM 9993 am 23. Mai 1995 hinterlegt.

Nachstehend wird eine erfindungsgemäße DNA in Form einer cDNA beschrieben. Diese steht beispielhaft für jede unter die vorliegende Erfindung fallende DNA.

Zur Herstellung einer erfindungsgemäßen cDNA ist es günstig, von einer Cosmid-Bibliothek, z.B. q1Z (vgl. Dietrich, A. et al., Nucleic Acids Res. 19, (1991), 2567-2572) auszugehen, von der Klone die Region Xq27.3-Xqter des menschlichen Genoms umfassen. Solche Klone werden auf einer Filtermembran fixiert und mit markierten, aus mRNA von Schweinegeweben, z.B. Gehirn, Muskel, Leber, durch reverse Transkription erhaltenen cDNA-Pools hybridisiert (vgl. Coy, J.F. et al., Mammalian Genome 5, (1994) 131-137). Diejenigen Klone, die mit den cDNA-Pools ein Hybridisierungssignal aufweisen, werden zum Screening einer menschlichen cDNA-Bibliothek, z.B. von fötalem Gehirngewebe, verwendet. Hierfür eignet sich besonders die cDNA-Bibliothek λ-Zap, Stratagene, Kat.-Nr. 936206. Es wird eine erfindungsgemäße cDNA erhalten.

Eine erfindunggemäße cDNA kann in einem Vektor bzw. Expressionsvektor vorliegen. Beispiele solcher sind dem Fachmann bekannt. Im Falle eines Expressionsvektors für E. coli sind dies z.B. pGEMEX, puC-Derivate, pGEX-2T, pET3b und pQE-8, wobei letzterer bevorzugt ist. Für die Expression. Hefe sind z.B. pY100 und Ycpadl zu nennen, während für die Expression in tierischen Zellen z.B. pKCR, pEFBOS, cDM8 und pCEV4, anzugeben sind. Für die Expression in Insektenzellen eignet sich besonders der Baculovirus-Expressionsvektor pAcSGHisNT-A.

Der Fachmann kennt geeignete Zellen, um eine, erfindungsgemäße, in einem Expressionsvektor vorliegende cDNA zu exprimieren. Beispiele solcher Zellen umfassen die E.coli-Stämme HB101, DH1, x1776, JM101, JM 109, BL21 und SG 13009, wobei letzterer bevorzugt ist, den Hefe-Stamm Saccharomyces cerevisiae und die tierischen Zellen L, 3T3, FM3A, CHO, COS, Vero und HeLa sowie die Insektenzellen sf9.

Der Fachmann weiß, in welcher Weise eine erfindungsgemäße cDNA in einen Expressionsvektor inseriert werden muß. Ihm ist auch bekannt, daß diese DNA in Verbindung mit einer für ein anderes Protein bzw. Peptid kodierenden DNA inseriert werden kann, so daß die erfindungsgemäße cDNA in Form eines Fusionsproteins exprimiert werden kann.

Des weiteren kennt der Fachmann Bedingungen, transformierte bzw. transfizierte Zellen zu kultivieren. Auch sind ihm Verfahren bekannt, das durch die erfindungsgemäße cDNA exprimierte Protein zu isolieren und zu reinigen. Ein solches Protein, das auch ein Fusionsprotein sein kann, ist somit ebenfalls Gegenstand der vorliegenden Erfindung.

Ein weiterer Gegenstand der vorliegenden Erfindung ist ein gegen ein vorstehendes Protein bzw. Fusionsprotein gerichteter Antikörper. Ein solcher Antikörper kann durch übliche Verfahren hergestellt werden. Er kann polyklonal bzw. monoklonal sein. Zu seiner Herstellung ist es günstig, Tiere, insbesondere Kaninchen oder Hühner für einen polyklonalen und Mäuse für einen monoklonalen Antikörper, mit einem vorstehenden (Fusions)protein oder Fragmenten davon zu immunisieren. Weitere "Booster" der Tiere können mit dem gleichen (Fusions)protein oder Fragmenten davon erfolgen. Der polyklonale Antikörper kann dann aus dem Serum bzw. Eigelb der Tiere erhalten werden. Für den monoklonalen Antikörper werden Milzzellen der Tiere mit Myelomzellen fusioniert.

Ein bevorzugter Antikörper der vorliegenden Erfindung, nämlich der monoklonale Antikörper 11 /78/1, wurde bei der DSM unter DSM ACC2211 am 26. April 1995 hinterlegt.

Die vorliegende Erfindung ermöglicht es, den Abbau chromosomaler DNA in apoptotischen Zellen zu untersuchen. Diese Untersuchung kann an isolierten Körperflüssigkeiten einer Person durchgeführt werden. Mit einem erfindungsgemäßen Antikörper kann eine für vorstehenden Abbau verantwortliche DNase nachgewiesen werden. Ferner kann mit einem erfindungsgemäßen Protein ein gegen diese DNase gerichteter Autoantikörper nachgewiesen werden. Beide Nachweise können durch übliche Verfahren, insbesondere einen Western Blot, einen ELISA, eine Immunpräzipitation oder durch lmmunfluoreszenz, erfolgen. Desweiteren kann mit einer erfindungsgemäßen Nukleinsäure, insbesondere einer DNA und hiervon abgeleiteten Primern, die Expression des für vorstehende DNase kodierenden Gens nachgewiesen werden. Dieser Nachweis kann in üblicher Weise, insbesondere in einem Southern Blot, erfolgen.

Darüberhinaus eignet sich die vorliegende Erfindung, Maßnahmen für oder gegen Apoptose, zu ergreifen. Diese Maßnahmen umfassen die Verabreichung eines erfindungsgemäßen Gegenstandes an eine Person. Mit einem erfindungsgemä-ßen Antikörper kann eine vorstehende DNase inhibiert werden, wodurch der Abbau von chromosomaler DNA verhindert wird. Andererseits kann mit einem erfindungsgemäßen Protein, insbesondere nach Kopplung an ein vom Körper nicht als fremd angesehenes Protein, z.B. Transferrin oder BSA, dieser Abbau gefördert werden, was sich insbesondere zur Behandlung von Tumorzellen eignen würde. Entsprechendes kann auch mit einer erfindungsgemäßen Nukleinsäure, insbesondere einer DNA, erreicht werden, die unter die Kontrolle eines in bestimmten Geweben, z.B. Tumoren, induzierbaren Promotors gestellt wird und nach ihrer Expression zur Bereitstellung eines erfindungsgemäßen Proteins in diesen Geweben führt. Darüberhinaus kann eine erfindungsgemäße Nukleinsäure, insbesondere eine DNA, auch zur Inhibierung einer vorstehenden DNase genutzt werden. Hierzu wird die Nukleinsäure, z.B. als Basis für die Erstellung von Anti-Sinn-Oligonukleotiden zur Expressions-Inhibierung der für vorstehende DNase kodierenden Gens verwendet.

Die vorliegende Erfindung stellt somit einen großen Beitrag zur diagnostischen und therapeutischen Erfassung von Apoptose dar.

### Kurze Beschreibung der Zeichnung:

- Fig. 1: zeigt die Basensequenz und die davon abgeleitete Aminosäuresequenz eines erfindungsgemäßen Proteins mit DNase-Aktivität.

Die vorliegende Erfindung wird durch die nachstehenden Beispiele erläutert.

### Beispiel 1: Herstellung und Reinigung eines erfindungsgemäßen Proteins

Zur Herstellung eines erfindungsgemäßen Proteins wurde die DNA von Fig. 1 als Template verwendet. Es wurde ein PCR-Verfahren durchgeführt. Als Primer-Paar wurde verwendet: 5'-CAGGGATCCGATGACGATGACAAAATGCACTACCCAAC TGCAC-3' und 5'-GGGGGATCCTCAGGCAGCAGGGCACAG-3'. Der PCR-Ansatz bzw. die PCR-Bedingungen waren wie folgt:

| **PCR-Ansatz** | |
|---|---|
| Temptate DNA (Fig. 1) | 1µl = 1 ng |
| Pfu-Polymerase 10x-Puffer | 10µl = 1 x |
| DMSO | 10µl = 10 % |
| dNTP's | 1µL = je 200µM |
| Oligonukleotide, je 1,5µl | 3µl = je 150 ng |
| H₂O-bidest | ad 99µl |

### PCR-Bedingungen

- 92°C - 5 min
- Zugabe von 1µl Pfu-Polymerase (Stratagene) = 2,5 Einheiten
- Zugabe von Paraffin

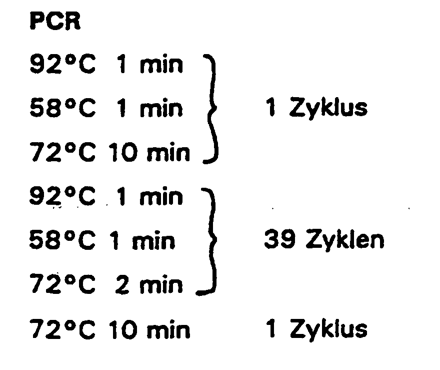

Die amplifizierte DNA wurde mit BamHl gespalten und in die einzige BamHI-Stelle des Expressionsvektors pQE-8 (Diagen) inseriert. Es wurde das Expressionsplasmid pQ/DNaseX erhalten. Dieses kodiert für ein Fusionsprotein aus 6 Histidin-Resten (N-Terminuspartner) und dem erfindungsgemäßen Protein von Fig. 1 (C-Terminuspartner). pQ/DNaseX wurde zur Transformation von E.coli SG 13009(vgl. Gottesman, S. et al., J. Bacteriol. 148, (1981), 265-273) verwendet. Die Bakterien wurden in einem LB-Medium mit 100µg/ml Ampicillin und 25µg/ml Kanamycin kultiviert und 4 h mit 60µM Isopropyl-β-D-Thiogalactopyranosid (IPTG) induziert. Durch Zugabe von 6 M Guanidinhydrochlorid wurde eine Lyse der Bakterien erreicht, anschließend wurde mit dem Lysat eine Chromatographie (Ni-NTA-Resin) in Gegenwart von 8 M Harnstoff entsprechend der Angaben des Herstellers (Diagen) des Chromatographie-Materials durchgeführt. Das gebundene Fusionsprotein wurde in einem Puffer mit pH 3,5 eluiert. Nach seiner Neutralisierung wurde das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).

Es zeigte sich, daß ein erfindungsgemäßes (Fusions)protein in hochreiner Form hergestellt werden kann.

### Beispiel 2: Herstellung eines erfindungsgemäßen Antikörpers

Ein erfindungsgemäßes Fusionsprotein von Beispiel 1 wurde einer 18 % SDS-Polyacrylamid-Gelelektrophorese unterzogen. Nach Anfärbung des Gels mit 4 M Natriumacetat wurde eine 35 kD Bande aus dem Gel herausgeschnitten und in Phosphat gepufferter Kochsalzlösung inkubiert. Gel-Stücke wurden sedimentiert, bevor die Proteinkonzentration des Überstandes durch eine SDS-Polyacrylamid-Gelelektrophorese, der eine Coomassie-Blau-Färbung folgte, bestimmt wurde. Mit dem Gel-gereinigten Fusionsprotein wurden Tiere wie folgt immunisiert:

### Immunisierungsprotokoll für polyklonale Antikörper im Kaninchen

Pro Immunisierung wurden 35µg Gel-gereinigtes Fusionsprotein in 0,7 ml PBS und 0,7 ml komplettem bzw. inkomplettem Freund's Adjuvans eingesetzt.

| | |
|---|---|
| Tag 0 | 1. Immunisierung (komplettes Freund's Adjuvans) |
| Tag 14 | 2. Immunisierung (inkomplettes Freund's Adjuvans: icFA) |
| Tag 28 | 3. Immunisierung (icFA) |
| Tag 56 | 4. Immunisierung (icFA) |
| Tag 80 | Ausbluten |

Das Serum des Kaninchens wurde im Immunoblot getestet. Hierzu wurde ein erfindungsgemäßes Fusionsprotein von Beispiel 1 einer SDS-Polyacrylamid-Gelelektrophorese unterzogen und auf ein Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wurde das Nitrocellulosefilter eine Stunde bei 37°C mit einem ersten Antikörper inkubiert. Dieser Antikörper war das Serum des Kaninchens (1:10000 in PBS). Nach mehreren Waschschritten mit PBS wurde das Nitrocellulosefilter mit einem zweiten Antikörper inkubiert. Dieser Antikörper war ein mit alkalischer Phosphatase gekoppelter monoklonaler Ziege Anti-Kaninchen-lgG-Antikörper (Dianova) (1:5000) in PBS. Nach 30-minütiger Inkubation bei 37°C folgten mehrere Waschschritte mit PBS und anschließend die alkalische Phosphatase-Nachweisreaktion mit Entwicklerlösung (36µM 5' Bromo-4-chloro-3-indolylphosphat, 400µM Nitroblau-tetrazolium, 100mM Tris-HCl, pH 9.5, 100 mM NaCl, 5 mM MgCl₂) bei Raumtemperatur, bis Banden sichtbar waren.

Es zeigte sich, daß erfindungsgemäße, polyklonale Antikörper hergestellt werden können.

### Immunisierungsprotokoll für polyklonale Antikörper im Huhn

Pro Immunisierung wurden 40µg Gel-gereinigtes Fusionsprotein in 0,8 ml PBS und 0,8 ml komplettem bzw. inkomplettem Freund's Adjuvans eingesetzt.

| | |
|---|---|
| Tag 0. | 1. Immunisierung (komplettes Freund's Adjuvans) |
| Tag 28: | 2. Immunisierung (inkomplettes freund's Adjuvans; icFA) |
| Tag 50: | 3. Immunisierung (icFA) |

Aus Eigelb wurden Antikörper extrahiert und im Western Blot getestet. Es wurden erfindungsgemäße, polyklonale Antikörper nachgewiesen.

### lmmunisierungsprotokoll für monoklonale Antikörper der Maus

Pro Immunisierung wurden 12µg Gel-gereinigtes Fusionsprotein in 0,25 ml PBS und 0,25 ml komplettem bzw. inkomplettem Freund's Adjuvans eingesetzt; bei der 4. Immunisierung war das Fusionsprotein in 0,5 ml (ohne Adjuvans) gelöst.

| | |
|---|---|
| Tag 0. | 1. Immunisierung (komplettes Freund's Adjuvans) |
| Tag 28: | 2. Immunisierung (inkomplettes Freund's Adjuvans; icFA) |
| Tag 56: | 3. Immunisierung (icFA) |
| Tag 84: | 4. Immunisierung (PBS) |
| Tag 87: | Fusion |

Überstände von Hybridomen wurden im Western Blot getestet. Erfindungsgemäße, monoklonale Antikörper wurden nachgewiesen. Einer davon, 11/78/1, wurde bei der DSM unter DSM ACC 2211 am 26. April 1995 hinterlegt.

### Beispiel 3: Nachweis der DNase-Aktivität eines erfindungsgemäßen Proteins

Es wurde ein DNase-Aktivitätstest entsprechend des Verfahrens von Rosenthal, A.L. & Lacks, S.A., Anal. Biochem. 80, (1977), 76-90 mit Modifikationen durchgeführt. Hierzu wurde ein 18 % SDS-Polyacrylamid-Gel hergestellt, das 2mM EDTA und denaturierte Lachs-Testis DNA oder Hefe RNA bis zu einer Endkonzentration von 10 µg/ml im Trenn- und Sammelgel enthielt. Proben wurden denaturiert, indem sie 4 min in Laemmli-Probenpuffer, der 5 % 2-Mercaptoethanol enthielt, gekocht wurden. Als Proben wurde ein erfindungsgemä-ßes Protein (von Beispiel 1) und Rinder-DNase 1 (Kontrolle) verwendet. Als Proteinmarker wurde eine 10 kd Leiter (Gibco BRL) verwendet, die in dem gleichen Gel aufgetrennt wurde, nach der Elektrophorese ausgeschnitten und mit Coomassie-Blau angefärbt wurde. Zur Entfernung des SDS nach der Elektrophorese wurde das die Proben enthaltende Gel 4 x 30 min mit 100 ml 40mM Tris-HCl, pH 7,6, gewaschen und über Nacht bei Raumtemperatur in 40 mM Tris-HCl, pH 7,6, mit 0,02 % Natriumazid und 2 mM MgCl₂, 2mM CaCl₂ bzw. mit 2mM MgCl₂, 2 mM ZnCl₂ inkubiert. Zum Nachweis der enzymatischen Aktivität wurde der Puffer gewechselt und Ethidiumbromid bis zu einer Endkonzentration von 2µg/ml hinzugegeben. Das Gel wurde periodisch auf einem langwelligen UV-Licht untersucht und photographiert.

Es zeigte sich, daß ein erfindungsgemäßes Protein eine DNase-Aktivität aufweist.

## Patentansprüche

1. Protein mit DNase-Aktivität, umfassend die Aminosäuresequenz von Fig. 1 oder ein funktionelles Derivat oder Fragment davon, wobei dieses Derivat oder Fragment (i) die Aminosäuresequenz von Fig. 1 mit Addition und/oder Substitution und/oder Deletion einer oder mehrerer Aminosäuren aufweist, (ii) von einer DNS codiert wird, die mit der DNS von Fig. 1 bei 20°C unter dem Schmelzpunkt der DNS hybridisiert, und (iii) eine DNase-Aktivität aufweist.

2. DNS, kodierend für ein Protein nach Anspruch 1.

3. DNS nach Anspruch 2, wobei die DNS umfasst:
(a) die DNS vom Fig. 1,
(b) eine bei 20°C unter dem Schmelzpunkt der DNS mit der DNS von (a) hybridisierende DNS oder
(c) eine mit der DNS von (a) oder (b) Ober den degenerierten genetischen Code verwandte DNS.

4. Expressionsplasmid, umfassend die DNS nach Anspruch 2 oder 3.

5. Transformierte Zelle enthaltend das Expressionsplasmid nach Anspruch 4.

6. Verfahren zur Herstellung des Proteins nach Anspruch 1, umfassend die Kultivierung der transformierten Zellen nach Anspruch 5 unter geeigneten Bedingungen.

7. Monoklonaler Antikörper, gerichtet gegen das Protein nach Anspruch 1.

8. Verwendung des Proteins nach Anspruch 1 zur Herstdlung eines Reagens zur Diagnose und/oder Therapie von Apoptose.

9. Verwendung der DNS nach Anspruch 2 oder 3 zur Herstellung eines Reagens zur Diagnose und/oder Therapie von Apoptose.

10. Verwendung des Antikörpers nach Anspruch 7 zur Herstellung eines Reagens zur Diagnose und/oder Therapie von Apoptose.

## Claims

1. Protein with DNase-activity, comprising the amino acid sequence of fig. 1 or a functional derivative or fragment thereof, wherein that derivative or fragment (i) comprises the amino acid sequence of figure 1 with addition and/or substitution and/or deletion of one or several amino acids, (ii) is coded by a DNA which hybridizes with the DNA of figure 1 at 20°C under the melting point of the DNA, and (iii) shows DNase-activity.

2. DNA encoding a protein according to claim 1

3. DNA according to claim 2, comprising:
(a) the DNA of figure 1,
(b) a DNA which hybridizes at 20°C under the melting point of the DNA with the DNA of (a) or
(c) a DNA that is related via the degenerated genetic code to a DNA of (a) or (b).

4. Expression plasmid comprising the DNA of claim 2 or 3.

5. Transformed cell containing the expression plasmid of claim 4.

6. Method for manufacturing the protein according to claim 1, that method comprising the cultivation under suitable conditions of the transformed cell of claim 5.

7. Monoclonal antibody, that is directed against the protein of claim 1.

8. Use of the protein according to claim 1 for manufacturing a reagent for the diagnosis and/or therapy of apoptosis.

9. Use of the DNA of claim 2 or 3 for manufacturing a reagent for the diagnosis and/or therapy of apoptosis.

10. Use of the antibody of claim 7 for manufacturing a reagent for the diagnosis and/or therapy of apoptosis.

## Revendications

1. Protéine avec une activité de désoxyribonucléase, comprenant la séquence d'acides aminés de la figure 1 ou un dérivé fonctionnel ou un fragment de cette séquence, ce dérivé ou ce fragment (i) présentant la séquence d'acides aminés de la figure 1 avec addition et/ou substitution et/ou délétion d'un ou plusieurs acides aminés, (ii) étant codé par un ADN qui s'hybride avec l'ADN de la figure 1 à 20 °C en dessous du point de fusion de l'ADN, et (iii) présentant une activité de désoxyribonucléase.

2. ADN codant pour une protéine selon la revendication 1.

3. ADN selon la revendication 2, l'ADN comprenant :
(a) l'ADN de la figure 1,
(b) un ADN s'hybridant à 20 °C en dessous du point de fusion de l'ADN avec l'ADN de (a), ou
(c) un ADN parent de l'ADN de (a) ou (b) par le code génétique dégénéré.

4. Plasmide d'expression comprenant l'ADN selon la revendication 2 ou 3.

5. Cellule transformée, contenant le plasmide d'expression selon la revendication 4.

6. Procédé pour la fabrication de la protéine selon la revendication 1, comprenant la culture de la cellule transformée selon la revendication 5 dans des conditions appropriées.

7. Anticorps monoclonal dirigé contre la protéine selon la revendication 1.

8. Utilisation de la protéine selon la revendication 1 pour la fabrication d'un réactif pour le diagnostic eUou la thérapie de l'apoptose.

9. Utilisation de l'ADN selon la revendication 2 ou 3 pour la fabrication d'un réactif pour le diagnostic et/ou la thérapie de l'apoptose.

10. Utilisation de l'anticorps selon la revendication 7 pour la fabrication d'un réactif pour le diagnostic et/ou la thérapie de l'apoptose.
